# EUROPEAN PATENT APPLICATION

(11) **EP 1 647 233 A1**
(43) Date of publication of application: **19.04.2006**
(21) Application number: 05256423.4
(22) Date of filing: 14.10.2005
(51) Int. Cl.: A61B 17/34, A61B 1/00, A61M 29/00

(54) **Transparent dilator device**

(30) Priority: 15.10.2004 US 966375
(71) Applicant: ETHICON ENDO-SURGERY, INC., Cincinnati, Ohio 45242 (US)
(72) Inventor: Bakos, Gregory J., Mason, Ohio 45040 (US); Bally, Kurt R., Lebanon, Ohio 45036 (US)
(74) Representative: Tunstall, Christopher Stephen

(57) **Abstract**

A medical device for use with an endoscope is provided. The medical device can include a tube with a first channel for receiving the endoscope. A transparent segment can extend from the distal end of the tube, and the transparent segment can have a first outer diameter sized for providing dilation of a portion of a body lumen. The medical device can also include a tapered tip attached to the distal end of the transparent segment that narrows from the first outer diameter to a distal terminating end. The endoscope is used to view the body lumen through the medical device while in use to dilate a stricture under direct visualization.

## Description

This application claims priority to and incorporates by reference US Serial Number 10/324,597 filed December 20, 2002.

### Field of the Invention

The present invention relates to a transparent dilator device for use in a lumen of a patient's body.

### Background

Constriction of natural body lumens, such as those of the human gastrointestinal tract, can occur in numerous ways. Some strictures are caused by muscular spasm, others by disease, and others by injury. Regardless of the cause, the typical method of treatment is to physically dilate the region using a medical device designed for that purpose.

Several types of devices are used for dilation. One generally established type is a bougie. Bougie tubes may be in the form of a mercury- or tungsten-filled tube with a tapered end that gradually opens the strictured esophagus as it is pushed past the treatment site. These devices come in a series of increasing sizes, each tube having a single effective dilating diameter, generally between 10 and 60 French. (French is a measure of circumference based on the diameter in millimeters, mm.) The bougie is typically introduced blindly after the physician has judged the proper beginning size with an endoscope. Some physicians follow a rule of thumb not to dilate a stricture more than three successive French sizes (3 mm) in a single session. If the lumen has not satisfactorily been opened after three sizes, the patient returns at a later time for another treatment session.

Another type of device is a wire-guided dilator. These devices are passed into the patient over a guidewire that has been pre-fed along a lumen of the gastrointestinal tract. The guidewire keeps the tip of the device in the lumen while it is being passed, to avoid perforating through the wall of the lumen. These devices have a single outer dilating diameter and typically have a radiopaque component so that they are visible under fluoroscopy. The following patent documents disclose various devices in the art: US 5,366,471; US 6,334,863; US 5,766,202; and GB 2023009A.

A third type of dilating device is a balloon. Balloon dilators may be comprised of polyethylene, and may be introduced through the working channel of an endoscope. The physician views the proximal end of a stricture site with an endoscope and introduces the deflated balloon into the narrowed area. The balloon is then inflated with saline or other fluid to effectively open the stricture site pneumatically. Balloons provide the advantages of multiple dilator diameters with a single intubation, passage through the working channel of an endoscope, and visualization of a stricture site from the proximal end.

Other devices such as double tapered bougies, pneumatic bougies, illuminating bougies, solid dilator devices attached to the distal end of an endoscope, and variable stiffness dilators have been previously described in the art. The following patent documents disclose various types of devices in the art: US 6,010,520; US 4,832,691; US 5,624,432; US 5,718,666; WO 98/47422.

### Summary of the Invention

Applicants have recognized the need for a relatively low cost dilator (disposable or non-disposable) which is relatively easy and convenient to use, and which allows the physician to directly visualize the stricture being dilated. In one embodiment, the present invention provides a medical device for use in dilating a body lumen. The medical device comprises at least one generally transparent segment, and a channel associated with the transparent segment for receiving a visualization device. The transparent segment includes at least one outer surface having a diameter sized for providing dilation. In one embodiment, the device can comprise a plurality of transparent sections, each section having an outer surface portion with a different diameter sized for sequential dilation of a stricture. The medical device can include at least one marking associated with the transparent segment for indicating the position and/or value of a dilation diameter.

The present invention also provides a method for dilating a portion of a body lumen. In one embodiment, the method can comprise the steps of: providing a medical device having at least one outer surface portion sized for providing dilation of a body lumen; inserting said outer surface portion into said lumen; advancing said outer surface portion in said lumen to dilate said lumen; and viewing dilation of said lumen through said outer surface portion of said medical device, such as by viewing the body lumen with a visualization device positioned within a channel in the medical device. The step of viewing dilation can comprise viewing the lumen through a transparent segment of device, wherein the transparent segment includes multiple outer diameters sized and arranged for sequential dilation.

### Brief Description of the Drawings

While the novel features of the invention are set forth with particularity in the appended claims, the invention in all its embodiments may be more fully understood with reference to the following description and accompanying drawings.
FIG. 1 is a side view of wire-guided dilator 10, described in prior art, including a guidewire channel 13, a first radiopaque marker 14, and a second radiopaque marker 15.
FIG. 2 is a side view of a first embodiment of the present invention, a dilator 18 which includes a handle 19, a tube 20, a transparent segment 21, and a tapered tip 22.
FIG. 3 is a sectional view of dilator 18 from FIG. 2 with an endoscope 50 inside a body lumen 60, proximal to a stricture 61.
FIG. 4 is a detailed view of transparent segment 21 and tapered tip 22 shown in FIG. 2, including a first outer diameter D1, a conical inner contour 32 inside tapered tip 22, an exterior taper angle thetal, and a narrow leading edge 34.
FIG. 5 shows a detail view of a preferred embodiment of transparent segment 21, including a second outer diameter D2, a third outer diameter D3, a plurality of markings 43, and at least one transition 44.
FIG 6 shows endoscope 50 inside a cross section view of dilator 18 shown in FIG 5, taken at line 6-6, including a field of view 52.

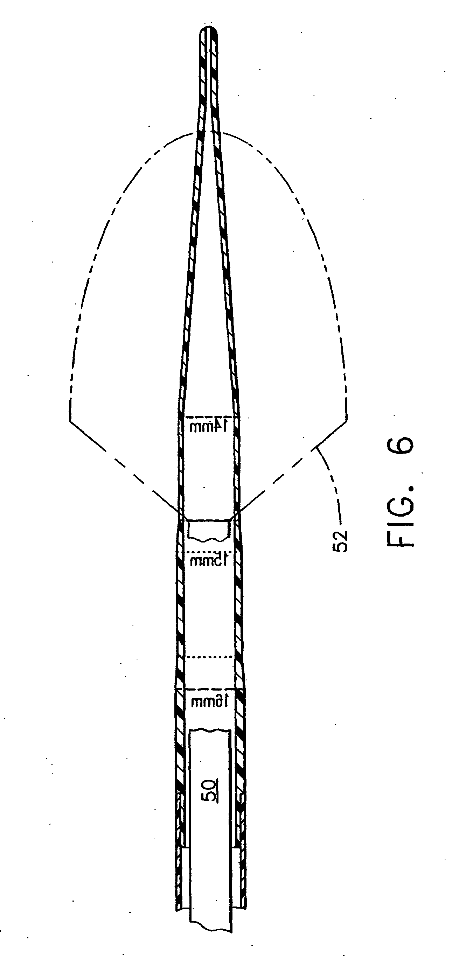

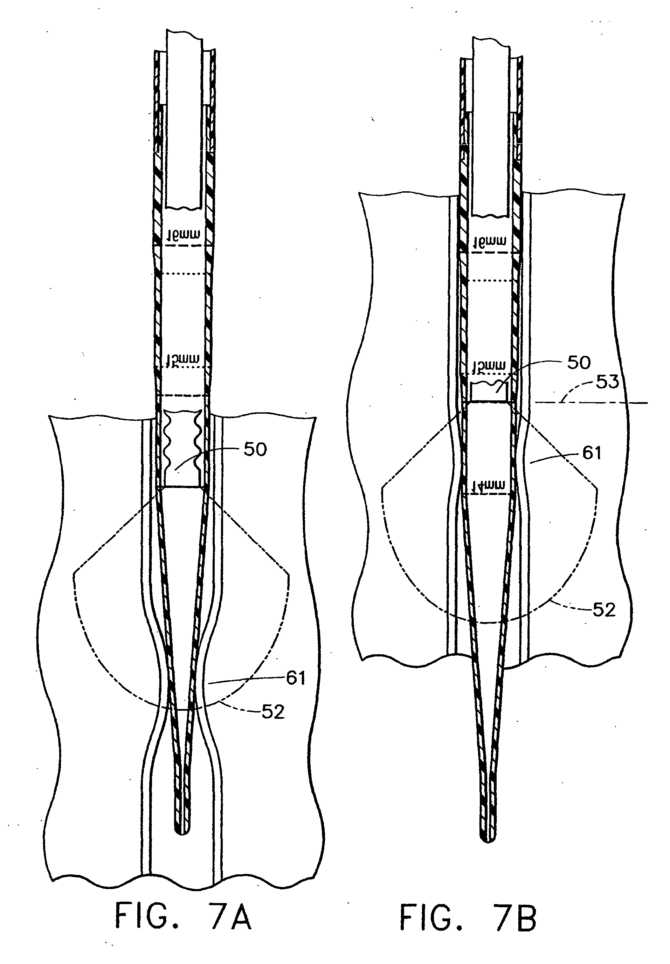

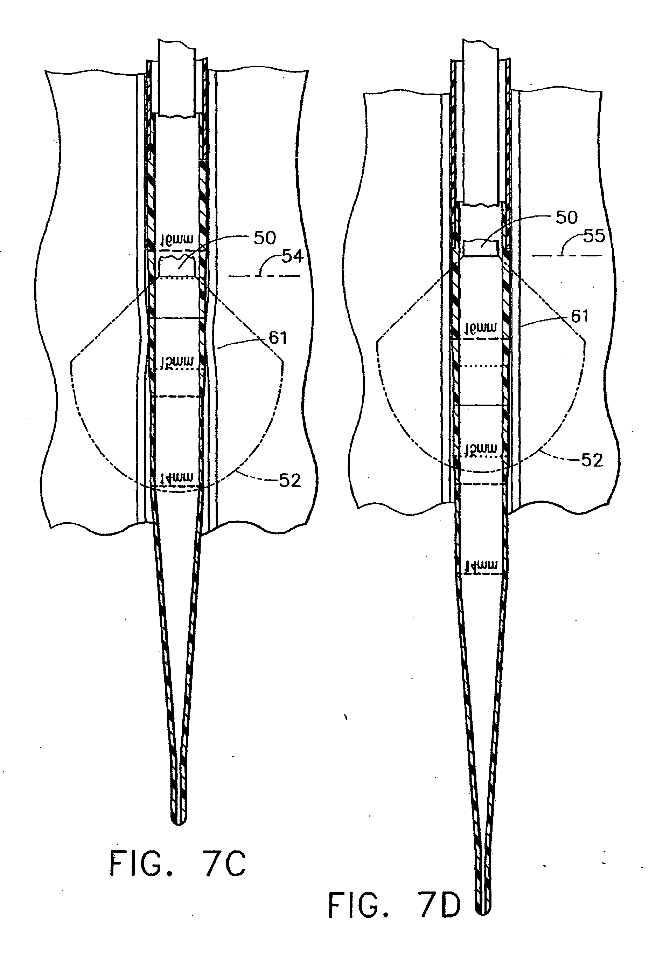

FIGs. 7A-7D illustrate steps which can be performed in using dilator 18 with an endoscope 50. FIG. 7A) is a sectional view of dilator 18 from FIG. 5 and field of view 52 of endoscope 50 positioned for advancement into body lumen 60 to a stricture 61. FIG. 7 B) is a sectional view of dilator 18 from FIG. 5 and field of view 52 of endoscope 50 positioned at first viewpoint 53 dilating to first outer diameter D1. FIG. 7 C) is a sectional view of dilator 18 from FIG. 5 and field of view 52 of endoscope 50 positioned at second viewpoint 54 dilating to second outer diameter D2. FIG. 7 D) is a sectional view of dilator 18 from FIG. 5 and field of view 52 of endoscope 50 positioned at third viewpoint 55 dilating to third outer diameter D3.
FIG. 8 shows one embodiment of dilator 18 having an internal channel having a tapered inner surface and having a decreasing inner diameter as the internal channel extends distally through one or more dilation segments.

### Detailed Description of the Invention

The present invention relates to the field of medicine, specifically to surgery, urology, or gastroenterology, in which a physician intends to alter the size of a constricted body lumen in a patient, or otherwise temporarily or permanently enlarge a portion of a body lumen. By way of example, the present invention is illustrated and described for application to an esophageal stricture of a human patient. However, the present invention is applicable for use in other natural lumens of human patients, including the urinary tract, biliary tract, lower gastrointestinal tract, or bronchus; and the present invention may also be used in other animals (e.g. for veterinary medicine), including mammals other than humans.

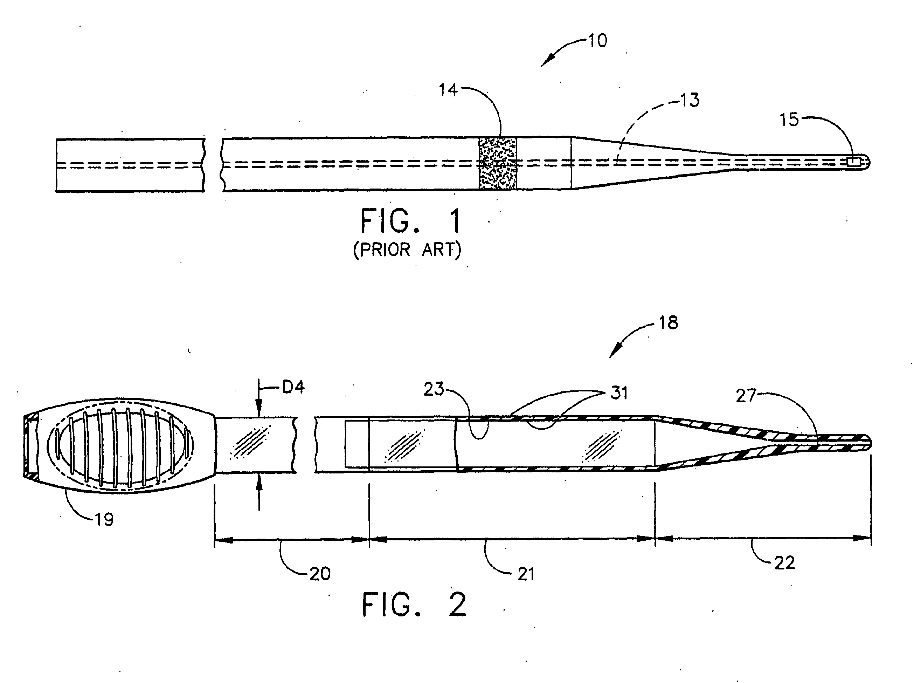

FIG. 1 shows a wire-guided dilator 10 described in prior art, including a guidewire channel 13, a first radiopaque marker 14, and a second radiopaque marker 15. Guidewire channel 13 allows wire-guided dilator 10 to be passed over a previously placed guidewire along the lumen of a patient. First radiopaque marker 14 and second radiopaque marker 15 are detectable under fluoroscopy to determine the position of wire-guided dilator 10 relative to a strictured area. This provides confirmation to the physician of dilation to the full diameter of the device. The fluoroscopy procedure can be costly and can expose the physician and patient to radiation.

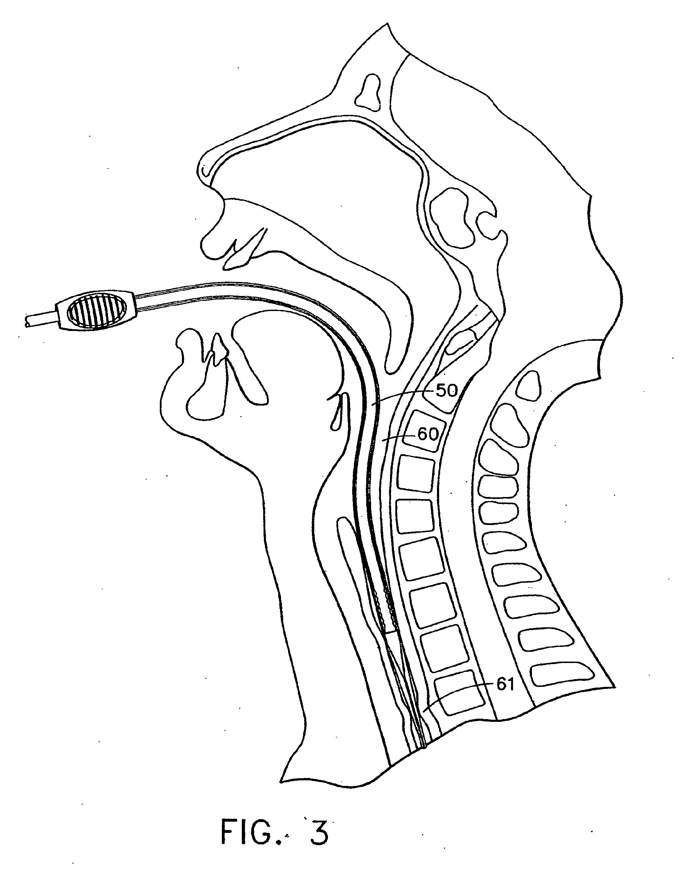

FIG. 2 shows a dilator 18 of the present invention, including a handle 19 at the device's proximal end, a tube 20, a transparent segment 21, and a tapered tip 22 at its distal end. Handle 19 of FIG. 2 includes a longitudinal channel for receiving an endoscope 50 (see FIG 3). Handle 19 provides the physician a location to grip dilator 18 and may be made from an elastic material, such as silicone or santoprene, an example of which is available from LNP Engineering Plastics, Inc. (Thorndale, PA) product code Colorcomp Santoprene 281-55 GYO 596-2. In one embodiment, the proximal portion of handle 19 can have an opening (such as an opening in a flexible, elastic seal or boot made of silicone, santoprene, or a suitable flexible elastic polymeric material), the hole being slightly smaller than the diameter of the endoscope. Such an arrangement can provide frictional engagement of the handle 19 with the endoscope 50 due to the drag force encountered in passing the endoscope through the opening. This feature allows the physician to hold either endoscope 50 or tube 20 in a one-handed fashion to position both the endoscope and the dilator 18 with direct visualization of a body lumen 60 (FIG. 3) during introduction or advancement of the device. In an alternate embodiment, a proximal opening in handle 19 may be slightly larger than the diameter of the endoscope to allow space for a guidewire or other medical accessory to reside alongside of the endoscope within a first channel 23 of tube 20. A guidewire may be employed to facilitate use of dilator 18 within the body.

Tube 20 shown in FIG. 2 may be made of a flexible polymer, examples of which include polyvinyl chloride (PVC), thermoplastic elastomer (TPE), polyurethane, or silicone. In one embodiment, tube 20 is made of a transparent flexible polymer, but it may also be made from an opaque material. Suitable transparent materials from which tube 20 can be manufactured include product number 2222RX-70 Clear 000X from Alpha-Gary Corporation (Leominster, MA) or clear flexible PVC available as 7777G-02 from Colorite Polymers (Ridgefield, NJ), among others. Commercially available clear flexible PVC tubing such as Kuri Tec K050 0810 from Kuriyama of America Inc. (Elk Grove Village, IL) may also be used for tube 20.

First channel 23 of FIG. 2 is appropriately sized to receive endoscope 50 (see FIG. 3). Various endoscopes of varying types and sizes may be used inside the present invention, including, but not limited to, bronchoscopes, colonoscopes, cystoscopes, and gastroscopes. Endoscope 50 may comprise a fiberscope or a videoscope, or may employ a CMOS (Complimentary Metallic Oxide Semiconductor) chip, a miniature camera, or other visualization device. Such a camera or visual system may be integral to the device, or may be a separate system. In one embodiment, first channel 23 may be about 2mm greater in diameter than the diameter of endoscope 50 used in the procedure so that relative motion between endoscope 50 and dilator 18 can occur smoothly under physician control. By way of example, a 9.5mm diameter gastroscope could be inserted into an 11.5mm diameter first channel 23 of dilator 18 to dilate an esophageal stricture. However, dilator 18 can be sized and configured to accommodate other endoscopes. In various other embodiments, first channel 23 may have a diameter in the range of, but not limited to, about 3mm to about 15mm.

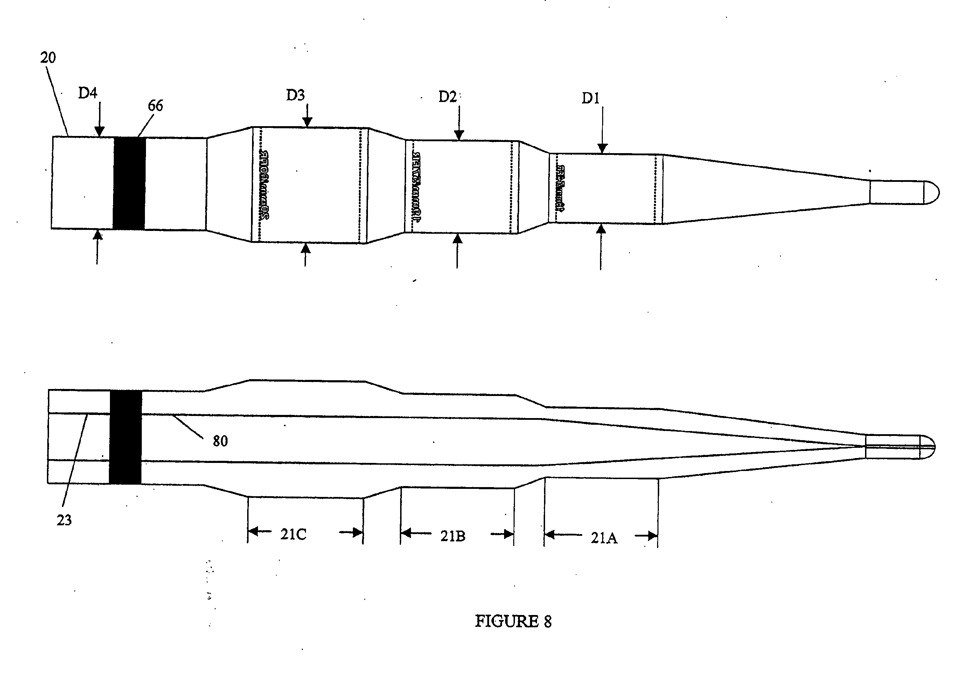

The outer diameter of tube 20 may have a diameter D4 shown in FIG 2. The size of diameter D4 is preferably made as small as feasible for reason of patient tolerance during the procedure. Diameter D4 may be equal to the largest diameter of transparent segment 21, or alternatively, D4 may be less than the largest diameter in transparent segment 21, as shown in FIG 8. A range of appropriate outer diameters for D4 include 3.5mm to 30mm, and more specifically 15mm to 20mm for embodiments designed for esophageal dilation.

The outer diameter D4 and channel 23 determine the range of wall thicknesses for tube 20. This wall thickness should provide adequate axial stiffness to advance transparent segment 21 of dilator 18 past stricture 61 without kinking, when used in conjunction with endoscope 50. The presence of endoscope 50 inside tube 20 during advancement adds to the stiffness of dilator 18, and greatly reduces the likelihood of kinking or folding tube 20 during use. In an embodiment made from a flexible PVC polymer designed for dilation in the esophagus, tube 20 may have a wall thickness from about 1mm to about 4mm, and more preferably between about 1mm to 2mm. A suitable polymer for tube 20 may have a range of durometers between about 60 to about 80 on the Shore A scale. One specific flexible PVC from which to make tube 20 is product number 7777G-02 from Colorite Polymers (Ridgefield, NJ), having a durometer of 77 on the Shore A scale.

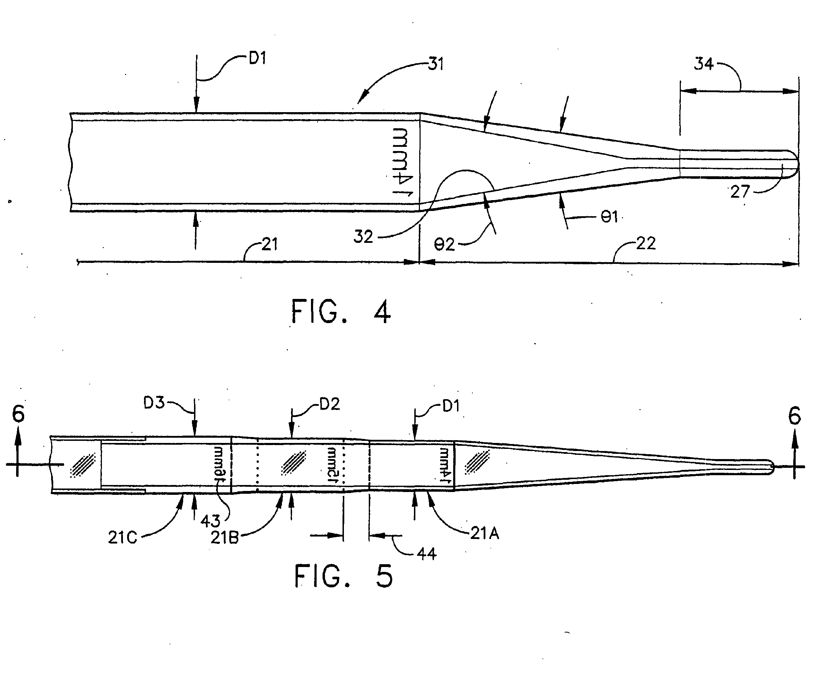

The length of tube 20 in FIG. 2 should be appropriately sized to comfortably reach the targeted area within the body while handle 19 remains outside the body for physician control. A low coefficient of friction on tube 20 allows easy sliding along the surface of body lumen 60. And, because the physician can reposition endoscope 50 within tube 20 to get a different view, easy sliding of endoscope 50 within tube 20 may also be desired. A suitable lubricant or low friction material (wet or dry) can be employed. For instance, a lubricating gel such as K-Y brand from Johnson & Johnson & Johnson may be used to lower the coefficient of friction between tube 20 and body lumen 60, and between endoscope 50 and tube 20. Likewise, a suitable lubricant or other low friction material (such as a coating 31 shown in Figure 4) could be applied to the inner or outer surfaces (or both) of tube 20 or transparent segment 21 (or both) to facilitate sliding of endoscope 50 within tube 20 and also sliding of tube 20 within body lumen 60.

FIG.3 shows the dilator 18 being used in body lumen 60 to dilate a stricture 61. Dilator 18 dilates stricture 61 in body lumen 60 under direct visualization by endoscope 50 positioned inside the device, allowing the physician to see along the entire length of stricture 61 from the inside out. The invention replaces the current methods of dilating body lumen 60 without direct visualization and improves on current methods of dilating with balloons and other devices that allow visualization from only the proximal side of stricture 61 during the procedure. By way of example, this illustration shows the invention used to dilate an esophageal stricture, but it could be used to dilate constrictions in other body lumens.

Transparent segment 21 shown in FIG. 4 and in FIG. 5 is a transparent portion of the device through which a physician views the stricture 61 during dilation of stricture 61. Flexible tip 22 can be unitary, single piece extension of transparent segment 21. Transparent segment 21 and flexible tip 22 can extend from the distal portion of tube 20 and may be made of a transparent material, including clear PVC, TPE, polyurethane, glass, or polycarbonate. Attachment means for attaching transparent segment 21 and flexible tip 22 to tube 20 may include a flange with adhesive, a plurality of mechanical ribs, a plurality of screw-type threads, or other combinations of geometric projections and adhesives. In one embodiment, attachment is provided by a heat bonding process achieved by closely aligning a distal end of tube 20 and a proximal end of transparent segment 21 on a mandrel, and simultaneously applying heat to melt the plastic while providing axial compression to join the parts, and holding them in place while they cool. Such a heat bonding process essentially forms tube 20 and transparent segment 21 into a single unitary piece. Alternatively, tube 20 and transparent segment 21 may also be formed as a single, unitary piece such as by forming tube 20 and transparent segment 21 together by molding or casting, which eliminates the need to join separate pieces into a single piece.

Transparent segment 21 can be constructed with an inner surface 80 (Figure 8) and one or more outer surfaces that are used to dilate a body lumen. The diameter of the outer surface is sized appropriately for the lumen that is being dilated. For example, a dilator for use with a gastroscope for esophageal strictures, outer diameters ranging from 10mm to 20mm may be appropriate, or if used for the colon, sizes ranging from 15mm to 30mm. For convenience, transparent segment 21 may be made with multiple (for example three) segments, each having an incrementally different diameter labeled D1, D2, and D3, such as shown in FIG. 5. For example, a "small" size esophageal dilator 18 may comprise a segment 21 A having diameter D1 of 14mm and a length measured axially of about 5mm to about 30mm, a segment 21 B having a diameter D2 of 15mm and a length measured axially of about 5mm to about 50 mm, and a segment 21C having a diameter D3 of 16 mm and a length measured axially of about 5mm to about 50 mm. A "medium" size dilator 18 may employ 16mm for D1, 17mm for D2, and 18mm for D3, and each segment having an axial length of about 5 mm to about 50 mm. A "large" dilator 18 may employ D1 of 18mm, a D2 of 19mm, and a D3 of 20mm, with the axial length of each segment being about 5mm to about 50 mm.

For applications with other types of endoscopes other than gastroscopes, other sizes of dilator 18 may be useful. For example, use with bronchoscopes that are smaller than gastroscopes may allow dilation of smaller strictures. Bronchoscopes are available in the range of 3.5mm OD to 6mm OD, and therefore dilator 18 may include outer surfaces with diameters from about 3.5mm to about 10mm. Therefore, dilator 18 may have outer dilating surfaces between about 3.5mm to about 30mm or larger, depending upon the type of endoscope placed inside.

Referring to the embodiment shown in Figure 8, the dilator 18 can have an inner surface 80 that is stepped or tapered to permit a relatively small outer Diameter D1 to be employed without sacrificing stiffness of the distal most segment 21 A. The stepped or tapered surface 80 provides a lumen of decreasing inner diameter as the surface 80 extends distally within one or more dilating segments. In Figure 8, surface 80 can have a generally constant inner diameter in segments 21C and 21B, and surface 80 can be tapered radially inward in segment 21A (the distal-most dilating segment). Such tapering can permit a dilator 18 formed from a single material and with a unitary construction to maintain a desired wall thickness and stiffness of the segment 21A even when the diameter D1 of segment 21A is approximately equal to the inner diameter of surface 80 through which the endoscope passes upstream of segment 21A. In the embodiment of Figure 8, the stepped or tapered inner surface 80 may prevent the endoscope from passing through the entire length of the distal most segment 21A. In this embodiment, the taper or step of surface 80 can be selected to permit the endoscope to advance to at least the proximal end of segment 21A before the endoscope abuts the inner surface 80.

In the embodiment shown in Figure 5, the wall thickness associated with each successive dilating segment decreases because the inner diameter of channel 23 is generally constant as the outer diameter reduces from D3 to D2 to D1. In the embodiment of Figure 8, the wall thickness may be maintained despite the reduction in outer diameter, so that weakening of the dilating segments (such as due to a wall thickness that is reduced in the distal dilating segment) is avoided, and so that the dilating segments can resist collapse due to radial pressure from the stricture being dilated. Accordingly, the embodiment of Figure 8 can be employed with a single piece construction and without using varying material properties to reinforce the distal most dilating segment.

FIG 8 shows one embodiment which maintains strength in the distal sections by gradually increasing the wall thickness of a distal segment (such as 21A) while maintaining its outer diameter constant at D1. Because of the increased wall in this section, inner surface 80 reduces from a diameter similar to channel 23 of Figure 5 (for passage of endoscope 50) to a diameter that is smaller than the diameter of the distal end of endoscope 50. Such an arrangement may be used when the outer diameter of distal segment 21 A is in the range of about 14mm to about 16mm, and inner surface 80 has an inner diameter of about 12mm proximal of segment 21A (such as in segments 21B and 21C and/or proximal of the dilating segments). An alternative method of maintaining radial strength is to form segment 21A from a harder durometer material to maintain the stiffness of the segment 21A when a relatively smaller wall thickness in segment 21A is employed.

Transparent segment 21 should be soft and flexible enough to be inserted into a body lumen without causing damage, but be stiff enough to break through a strictured area. Material selection and part geometry can both be used to achieve this balance of flexibility and radial stability. When using a flexible PVC material with a durometer of between 60 and 80 on the Shore A scale, a minimum wall thickness to maintain adequate radial strength of segment 21 is approximately 1.5mm. If a portion of transparent segment 21 is made from a relatively more rigid material (for example a durometer from 80 to 90 Shore A), a wall thickness less than 1.5mm may be used. Short rigid segments may also be incorporated into transparent segment 21, especially in instances where wall thickness is about 1mm or less. Such rigid segments could be over-molded in place or fixed by adhesive in a desired location. When used in the esophagus, the length of any rigid segment should be minimized to allow easy passage through the cricopharyngeal junction in the back of the throat. Rigid segments 25mm or less may be appropriate for esophageal dilation.

The transparency of transparent segment 21 allows direct visualization of tissue outside the device from endoscope 50 located within the device. Therefore, it can be desirable to minimize distortion or obstruction of view through transparent segment 21. Suitable transparency of transparent segment 21 can be accomplished by controlling material selection and molding finish. The material from which transparent segment 21 is constructed should be clear, and the mold used should be polished so that the molded part has a smooth outer surface. Transparent segment 21 may include markings 43 (Figure 5) such as one or more markings 43 indicating the outside diameter of transparent segment 21 at the longitudinal position of the particular marking. Anatomical landmarks, color variations, tissue differences, foreign bodies, and any markings 43 (FIG. 5), and other items of interest should be recognizable when viewed with endoscope 50 disposed within transparent segment 21. An appropriate material for transparent segment 21 can have a haze value of about 5% or less, and have a light transmission property of about 80% or greater. Haze value is a material property, expressed in percent, describing the amount of "cloudiness" in a material caused by particulate impurities, molecular structure, or degree of crystallinity, resulting in scattering of light and apparent cloudiness. Light transmission is a material property indicating the percentage of incident light that passes through an object. In addition to using transparent material to form segment 21, a mold used to create segment 21 can be highly polished to create a smooth surface that does not distort the view seen through endoscope 50 when the viewing device of endoscope is disposed inside segment 21 to view lumen tissue outside of segment 21.

Having a low coefficient of friction of transparent segment 21 allows the device to slide freely inside body lumen 60, especially during dilation of stricture 61. Lubricating gel, such as K-Y brand lubricating jelly available from Johnson and Johnson can be used to lower the coefficient of friction during use. In one embodiment, a coating 31 (FIG. 2) can be disposed on one or both of the inner and outer surfaces of the transparent segment 21, in which coating 31 is transparent and has a lower coefficient of friction when hydrated than when dry. One example of coating 31 is a hydrogel material made by the interaction of polyvinylpyrrolidone with one or more isocyanate prepolymers. A coating such as Hydromer® Lubricious Medical Coatings by Hydromer Inc. (Somerville, NJ) can be used for coating 31. Such a coating can reduce the drag force along the axis of a lumen during dilation, creating a more efficient device when compared to existing dilators.

FIG. 4 shows a section view of dilator 18 including a first outer diameter D 1 and a tapered tip 22. Tapered tip 22 may be made of a flexible polymer that is pliable compared to body tissue and may be attached to the distal end of transparent segment 21. In one embodiment, tapered tip 22 can be made with the same material as transparent segment 21, if transparent segment 21 is made of a flexible polymer. In one embodiment, a biomedical grade of clear flexible PVC having a hardness value of about 60 to 80 on the Shore A scale can be used to form tapered tip 22 and transparent segment 21. For example, a clear flexible PVC material such as XV-3450 from PolyOne Corp. (Avon Lake, OH) could be used to mold both transparent segment 21 and tapered tip 22 as a single part. Another material from which tip 22 and segment 21 may be formed is material designated 7077G-02 from Colorite Polymers (Ridgefield, NJ), which material can be gamma stable to allow gamma radiation to be used for sterilization. Other suitable materials such as TPE or polyurethane can also be used.

Tapered tip 22 facilitates intubation into body lumen 60 by gradually tapering from a first outer diameter D1 to a narrow leading segment 34 with an exterior taper angle thetal, as shown in FIG. 4. In one embodiment, tapered tip 22 includes a second channel 27 in communication with a conical inner contour 32 and channel 23 so that the device can be threaded over a guidewire. Second channel 27 can be sized appropriately for a guidewire, including diameters in the range from about 0.5mm to about 1.5mm. Exterior taper angle thetal can be selected to provide a desired amount of radial force transmitted against stricture 61 for a given level of axial force (force parallel to length of the dilator 18) applied by the physician. Generally, a low value of exterior taper angle provides an efficient, comfortable transmission of radial force against the stricture, with the trade-off that low values of exterior taper angle generally increase the length of the dilator 18 that must be inserted past the stricture. For instance and without limitation, the dilator can have an exterior taper angle thetal in a range including about 3 degrees to about 15 degrees. In one embodiment, the taper angle can be between about 6.5 and about 7.5 degrees.

FIG. 4 shows conical inner contour 32 connecting channel 23 to second channel 27 with an interior taper angle theta2. The connection has a conical shape to reduce the glare from endoscope 50 during use. Conical inner contour 32 also facilitates molding by allowing a central core pin to be tapered for ease of removal. In one embodiment, the value of interior taper angle theta2 is different from value of exterior taper angle thetal to provide a varying wall thickness along the length of the device, so that radial strength can be tailored as needed along the length of the device. By way of example, the exterior taper angle thetal can be about 7 degrees, and the interior taper angle theta2 can be about 6 degrees.

FIG. 5 depicts an embodiment of transparent segment 21 comprised of a first transparent section 21 A having a first outer diameter D1; a second transparent section 21B having a second outer diameter D2, and a third transparent section 21C having a third outer diameter D3, each pair of adjacent sections separated by a transition 44. The first, second and third sections can each be generally cylindrical. Transitions 44 provide a tapered (linear or curvilinear) transition in diameter from the outer diameter of one section to the outer diameter of the adjacent section. The transitions can have can have a hollow conical configuration, such as a conical shape generally the same as that of a truncated cone having a centrally located passageway. Alternatively, the dilator 18 can have diameters D1, D2, D3, and D4 on a single continuous outer surface portion which is tapered linearly or curvilinearly from D1 to D4.

Figure 5 also shows plurality of markings 43 for the physician to select and position the desired dilating diameter in the area of stricture 61 during the procedure. Markings 43 provided to be visible through the optical device (e.g. camera, fiber optic cable, etc.) associated with the endoscope 50 and may have several uses, including delineating the boundaries of a single dilating diameter, or indicating the numeric value of a dilating diameter. Markings 43 may be molded into the part, applied with ink, etched on the device, or applied by any other suitable method. In one embodiment, numerical indications may be applied to the outer surface of transparent segment 21 in multiple locations, some of which are readable by endoscope 50 from inside the device (necessitating them to appear backwards from the exterior of the device, but appearing forward from inside the device), and some of which are readable from the exterior of the device (appear backward from endoscope 50 inside the device). In addition to markings comprising numerals or letters, other embodiments of plurality of markings 43 may include use of various other indicia, including without limitation one or more different colors, and/or use of different geometric shapes, such as to designate different sections or segments, or attributes of different portions of the device. For instance, a row or column of circles could be used to designate a first section, a pattern of circles and dashes could designate a second section, and a pattern of circles, dashes, and squares could designate a third section. Markings 43 can also be coated or otherwise treated with a substance to make them luminous or glow in reduced lighting.

FIG. 6 shows a cross section of transparent segment 21 and tapered tip 22 of FIG 5 taken at line 6-6 with endoscope 50 positioned inside. A field of view 52 is depicted to indicate the area in view by the physician. Because endoscope 50 is movable with respect to dilator 18, a change in the position of endoscope 50 allows the physician to see a different area of body lumen 60 within field of view 52.

FIGs 7A-7D show four possible steps a physician may use to dilate stricture 61 with dilator 18 and endoscope 50. FIG. 7A shows the relative positions of endoscope 50 and dilator 18 upon insertion into body lumen 60 to a location of stricture 61. In this position, field of view 52 is used to view body lumen 60 during insertion, and to view the proximal location of stricture 61.

FIG 7B shows endoscope 50 at a first viewpoint 53 so field of view 52 includes first outer diameter D1. While viewing plurality of markings 43 for reference, dilator 18 is advanced into stricture 61 causing dilation to first outer diameter D1. Plurality of markings 43 may delineate the boundaries of diameter D1 and may also indicate its numerical value. In this manner, the physician has a visual indication through endoscope 50 of where to position dilator 18 with respect to stricture 61 for precise dilation to a desired diameter.

FIG. 7C shows a next potential step to further dilate stricture 61 if desired by the physician. Endoscope 50 is placed at a second viewpoint 54 relative to dilator 18 so that field of view 52 includes second outer diameter D2. The medical device is further advanced into stricture 61 to further dilate to second outer diameter D2 while viewing another portion of plurality of markings 43 for reference.

FIG. 7D shows endoscope 50 at a third viewpoint 55 so that field of view 52 includes third outer diameter D3. Again, the medical device may be further advanced to dilate stricture 61 to third outer diameter D3 while again viewing yet another portion of plurality of markings 43 as a reference. In this manner, the physician can visually examine the entire length of a stricture with endoscope 50 as the dilation occurs.

An alternative method of use is to first place a guidewire in body lumen 60 of the patient, then thread dilator 18 over that guidewire using second channel 27, conical inner contour 32, and channel 23. Dilator 18 may then slide into the guidewire, after which endoscope 50 may be placed into channel 23. The guidewire does not need to be threaded through the working channel of endoscope 50, but a physician may do so if desired. The combination of endoscope 50, dilator 18, and guidewire could then be used according to the steps illustrated in FIGs. 7A-7D.

A physician advancing a dilator through a stricture 61 will normally feel resistance. In prior art devices where the physician attempts to "blindly" introduce a dilator, perforation or other damage to the body lumen may occur. Further, if such damage occurs, the physician may not immediately recognize that damage has occurred. The present invention can permit the physician to visualize a medical procedure (e.g. dilation of a stricture) as the procedure is performed, thereby providing the physician with immediate feedback on the state of the tissue being treated. Such visualization can help in avoiding unintended damage of tissue which might otherwise occur if the physician is not able to directly visualize the procedure. In the unlikely even that damage does occur, the physician can immediately notice it and can choose to cease treatment and begin a new course of action to repair the damage. Direct visualization provided by endoscope 50 inside dilator 18 allows the physician to know that he/she has not perforated, bruised, or otherwise damaged body lumen 60.

Another useful feature of the dilator 18 is that it provides one with the ability to dilate to more than one diameter with a single introduction of the device, and with precision. This is made possible by the ability to see plurality of markings 43 from inside transparent segment 21 to identify a particular dilating diameter. Previously disclosed devices with multiple diameters rely on tactile feedback, remote markings, or costly pressure meters (e.g. in the case of balloons) to control the diameter. The intuitive nature of plurality of markings 43 allows the physician to easily select the desired diameter by looking clearly through the device, along the whole length of stricture 61, adding precision to the device when compared to current methods, which may involve elaborate measuring schemes.

Dilator 18 can also be less costly to manufacture than some balloon style dilators. Accordingly, devices of the present invention may be cost effectively packaged and sold as a single-use, disposable product which does not require cleaning or re-sterilization. Dilator 18 can be pre-sterilized and packaged in a sterile pouch or other suitable package.

Dilator 18 can also provide reliability in terms of dilating diameter compared to certain balloon type dilators. Some balloons may not hold a constant diameter when inflated, so the dilation is not as reproducible as a tube of known size being passed through a constricted area. Dilator 18 provides two-vector shearing of stricture 61. This results from sliding a tapered-tip device through a narrowed area, thereby applying forces in both the axial and radial directions. Balloons typically only apply a generally radial directed force to a stricture.

Dilator 18 can also provide affordable and convenient dilation with the ability to directly visualize the treatment along the entire length (not just a proximal or distal portion) of a stricture 61 without the use of expensive or potentially harmful radiographic equipment to confirm placement. When using radiographic equipment, a dilation procedure is typically performed in a radiographic suite, which often requires additional logistics of scheduling an additional appointment and different staffing needs, which in turn can require additional time and cost. Dilator 18 can provide complete direct visualization with endoscope 50 without the additional costs or time associated with radiographic equipment.

In one embodiment, the dilator can have the following construction and have the following dimensions. For example, tube 20 may have an inner diameter of 12.5mm and an outer diameter of 15.8mm and a length of about 55cm. This shaft may be constructed from clear flexible PVC, such as Colorite 7777G-015 having a Shore A hardness of 77 and attached to transparent segment 21 through a thermal bond process. The joint between tube 20 and transparent segment 21 may be covered with a visually identifiable mark 66 in FIG. 8, such as a dark band of ink. Such a band may be useful to a physician to indicate the beginning of the dilating region. A first "small" size dilator may include a transparent segment 21 comprising three dilating segments and be constructed from clear flexible PVC such as Colorite 8077G-015 with a Shore A hardness of 80. A distal dilating segment 21 A may comprise an outer diameter D1 of 14mm, an axial length of 25mm, and a wall thickness of about 1.75mm. A middle dilating segment 21 B may comprise an outer diameter D2 of 15mm, an axial length of 25mm, and a wall thickness of about 1.85mm. A proximal dilating segment 21C may comprise an outer diameter D3 of 16mm, an axial length of 25mm, and a wall thickness of about 2.1mm. Between each dilating segment is a 7 degree taper of about 9mm in axial length, and the tip of transparent segment 21 comprises a 7 degree taper of about 80cm in axial length that ends in a straight diameter of 4.5mm for an axial length of 25mm.

A second embodiment may comprise the same shaft, with a transparent segment constructed of Colorite 8077G-015 to form a "Medium" size with the following dimensions. A distal dilating segment 21 A may comprise an outer diameter D1 of 16mm, an axial length of 25mm, and a wall thickness of about 2.7mm. A middle dilating segment 21 B may comprise an outer diameter D2 of 17mm, an axial length of 25mm, and a wall thickness of about 2.7mm. A proximal dilating segment 21C may comprise an outer diameter D3 of 16mm, an axial length of 25mm, and a wall thickness of about 3mm. Between each dilating segment is a 7 degree taper of about 9mm in axial length, and the tip of transparent segment 21 comprises a 7 degree taper of about 95cm in axial length that ends in a straight diameter of 4.5mm for an axial length of 25mm.

A third embodiment may comprise the same shaft, with a transparent segment constructed of Colorite 7077G-015 having a Shore A hardness of 70 to form a "Large" size with the following dimensions. A distal dilating segment 21 A may comprise an outer diameter D1 of 18mm, an axial length of 25mm, and a wall thickness of about 3mm. A middle dilating segment 21 B may comprise an outer diameter D2 of 19mm, an axial length of 25mm, and a wall thickness of about 3.5mm. A proximal dilating segment 21C may comprise an outer diameter D3 of 20mm, an axial length of 25mm, and a wall thickness of about 3.9mm. Between each dilating segment is a 7 degree taper of about 9mm in axial length, and the tip of transparent segment 21 comprises a 7 degree taper of about 110cm in axial length that ends in a straight diameter of 4.5mm for an axial length of 25mm.

Without being limited by theory, a dilator formed of a material having a Shore A hardness of between about 60-90 and a wall thickness of between about 1.0mm and about 4.0 mm can provide flexibility for easy insertion, yet be rigid enough to provide dilation of strictures encountered in the body.

The dilator of the present invention can be used according to the following steps. As a preliminary step, an endoscope can be used to identify the stricture area and estimate the size of the dilator desired to treat the stricture area. If desired, a guidewire may first be inserted through the accessory channel of an endoscope into the stricture area. The endoscope can then be withdrawn while the position of the guidewire is maintained, with a proximal end of the guidewire extending outside the body. The dilator 18 of the present invention can then be removed from its sterile packaging. If a guidewire is to be used, the proximal end of the guidewire extending from the patient can inserted through channel 27 of tapered tip 22. A lubricant such as K-Y brand gel lubricant can be applied to the outside surface of the endoscope. The distal end of the endoscope can be inserted partially into the proximal end of the dilator, and lubricant such as K-Y brand gel can be applied to the exterior of the dilator 18. If a guidewire is used, rather than again feeding the guidewire through the accessory channel of the endoscope, the endoscope can be placed inside the dilator 18 along side of the guidewire. With the viewing optics of the endoscope disposed inside the dilator 18, the dilator 18 and endoscope can be intubated into the patient's body (e.g. esophagus) and advanced to the stricture site, while visualizing the intubation with the endoscope through the dilator.

The dilator 18 and endoscope can be advanced to dilate the stricture site in a step-wise fashion, and in each step the procedure can be viewed with the endoscope through the dilator. The endoscope can be positioned in the dilator such that the endoscope is used to view the body lumen through smallest desired dilating segment, such as the distal-most and smallest outer diameter dilating segment of the dilator. The diameter markings or other indicia (e.g. markings 43) on the dilator can be used to identify the desired dilating segment. Using the markings as a guide, the endoscope and dilator can be advanced together so that the stricture is viewed and positioned between markings provided on the dilator. To dilate to a larger diameter, the endoscope can be withdrawn proximally within the dilator 18 to be repositioned to view through the next larger dilating diameter of the dilator. Then the endoscope and dilator can again be advanced together so that the stricture is viewed and positioned between the next larger diameter, as indicated by the stricture being viewed as positioned between markings indicating the next diameter segment of the dilator. If necessary, the above procedure can be repeated to dilate to a third diameter. If desired, a dilator can be provided with four or more dilating segments. The procedure can be repeated for each dilating segment, if desired. Once dilation of the stricture is complete, as can be visually verified by viewing through the dilator of the present invention, the guidewire (if used) can be removed, the endoscope can be repositioned distally in the dilator to provide the most advantageous view through the distal tip of the dilator, and the endoscope and dilator can then be removed together, with the removal step viewable through the endoscope.

In prior dilation methods, the physician typically would rely at least partially on the "feel" of resistance in introducing a dilator to a stricture, and would typically follow a "rule of three" guideline to only dilate to two additional diameters once resistance is encountered. The present invention allows the physician to view the stricture being dilated as dilation occurs, to visually verify that dilation is occurring without perforation or other injury to the patient.

The present invention has been illustrated as having a transparent segment having generally circular cross-sections, but non-circular cross-sections (e.g. oval, elliptical, polygonal) can also be used, in which case the term "diameter" will be understood to refer to the maximum dimension of the non-circular cross-section used for providing dilation. The present invention may be provided in kit form with other medical devices, and the kit elements can be pre-sterilized and packaged in a sealed container or envelope to prevent contamination. The present invention may be provided as single use disposable device or alternatively, may be constructed for multiple uses.

While various embodiments of the present invention have been disclosed, it will be obvious to those skilled in the art that such embodiments are provided by way of example only. Further, each element or component of the present invention may be alternatively described as a means for performing the function or functions performed by the element or component. Numerous variations, changes, and substitutions will now occur to those skilled in the art without departing from the invention. Accordingly, it is intended that the invention be limited only by the spirit and scope of the appended claims.

## Claims

1. A medical device for use in dilating a body lumen, the medical device comprising at least one generally transparent segment, and wherein said transparent segment includes at least one outer surface having a diameter sized between about 3.5 mm and about 30 mm for providing dilation.

2. The medical device of Claim 1 wherein said transparent segment comprises a plurality of outer surface portions, each outer surface portion having a different diameter, and said diameters sized for providing sequential dilation of a stricture.

3. The medical device of Claim 2 wherein said transparent segment comprises at least three outer surface portions, each outer surface portion having a different diameter.

4. The medical device of Claim 1 wherein said transparent segment comprises at least one marking for indicating the position of a diameter sized for providing dilation.

5. The medical device of Claim 1 wherein said transparent segment comprises at least one marking for indicating the size of a diameter for providing dilation.

6. The medical device Claim 1 further comprising a channel associated with said transparent segment, said channel sized for receiving an endoscope, wherein said channel has an internal diameter of between about 3mm and about 15 mm.

7. The medical device of claim 1 wherein said transparent segment comprises a material having a hardness between 60 and 90 on the Shore A scale.

8. The medical device of claim 7 wherein at least one transparent segment has a wall thickness of between about 1.0 to about 3.5 mm.

9. A medical device for dilating strictures within the body, the device comprising:
a first transparent segment having an outer diameter between about 12mm and about 20mm; and
a second transparent segment having an outer diameter at least about 1mm greater than the outer diameter of the first transparent segment.

10. The medical device of Claim 9 comprising a third transparent segment having an outer diameter at least about 1 mm greater than the outer diameter of the second transparent segment.

11. A medical device for dilation of a body lumen comprising:
a transparent segment comprising an inner surface and at least one outer surface sized for dilation of a body lumen;
wherein a proximal portion of said inner surface is sized to pass an endoscope; and
wherein a distal portion of said inner surface is sized to be smaller than the endoscope.

12. A medical device for use with a flexible endoscope to dilate a stricture, the device comprising:
a flexible transparent section comprising an inner surface and at least one outer surface sized for dilation of a body lumen;
wherein the inner surface comprises a proximal portion having an inner diameter sized to pass an endoscope;
wherein the inner surface comprises a distal portion having an inner diameter smaller than that of the endoscope; and
wherein the outer surface has at least one generally constant diameter portion along the length of the transparent section.
